(19) Europäisches Patentamt
European Patent Office
Office européen des brevets

(11) **EP 4 309 573 A1**

(12) **EUROPEAN PATENT APPLICATION**

(43) Date of publication:
**24.01.2024 Bulletin 2024/04**

(21) Application number: **22186284.0**

(22) Date of filing: **21.07.2022**

(51) International Patent Classification (IPC):
**A61B 5/026** (2006.01)    **A61B 5/00** (2006.01)

(52) Cooperative Patent Classification (CPC):
**A61B 5/0261; A61B 5/6815; A61B 5/6817;
A61B 5/6824; A61B 5/6826; A61B 5/6833;
A61B 5/7207;** A61B 2562/0233

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Sonion Nederland B.V.
2132 LS Hoofddorp (NL)**

(72) Inventors:
• **Fransen, Alwin
2132 LS Hoofddorp (NL)**
• **Post, Peter Christiaan
2132 LS Hoofddorp (NL)**

(74) Representative: **Inspicos P/S
Agern Allé 24
2970 Hørsholm (DK)**

(54)    **DETERMINATION OF A PARAMETER RELATED TO BLOOD FLOW IN A BLOOD PERFUSED PART USING A VCSEL**

(57)    A system for determining a parameter related to blood flow of a blood perfused part of a user, such as of an ear canal, the system comprising a housing, one or more VCSEL(s) each with Integrated Photodetector and each being provided at or in the housing, the VCSEL(s) with Integrated Photodetector being positioned so as to emit radiation to and receive radiation from a surface of blood perfused part and a controller for receiving output signal(s) from the VCSEL(s) with Integrated Photodetector and determine the parameter related to the blood flow.

Figure 3

Processed by Luminess, 75001 PARIS (FR)

**Description**

The present invention relates to a system and a method for determining a parameter relating to blood flow in a blood perfused part, and more particularly to the use of VCSEL with integrated photodetectors for the measurement, where the parameter may be compensated for motion between the VCSEL(s) with integrated photodetector and the part and/or may be performed based on Doppler shifting.

Relevant technology may be seen in US2011/0105874, US7018338, US8979762, US8157730, US2010/081940, US2010/177300, US2021/196136, US2021/085245, US5711308, US2020/275216, US2020/370879, US2011/082355, EP3439550, WO18/029033, and US2008/0188726.

In a first aspect, the present invention relates to a system for determining a parameter related to blood flow of a blood perfused part of an ear canal, the system comprising:

- a housing configured to be provided in the ear canal,

- one or more VCSEL(s) each with Integrated Photodetector and each being provided at or in the housing, the VCSEL(s) with Integrated Photodetector being positioned so as to emit radiation to and receive radiation from a surface of the ear canal and

- a controller for receiving output signal(s) from the VCSEL(s) with Integrated Photodetector and determine the parameter related to the blood flow.

In the present context, a VCSEL is a Vertical-cavity surface-emitting laser and a VCSEL with integrated photodetector is a very compact element well suited for use in space critical situations. A Vertical-cavity surface-emitting laser, or VCSEL, is a laser having an optical cavity in which the radiation is created and from which the radiation is emitted. The built-in photodetector, which could be of any type, such as a photodiode, photo resistor, CCD, phototransistor or the like, is positioned so as to detect radiation, such as generated radiation, in the laser/optical cavity. The active region of the laser is provided in the optical cavity and may constitute the optical cavity. Two reflecting elements, often Bragg reflectors, are provided on opposite sides of the optical cavity or active region. Other optical components, such as lenses, slits, reflectors or the like, may be provided outside of the VCSEL. Clearly, the VCSEL with integrated Photodetector may itself be exposed to the surroundings of the system, such as if provided on the housing or in an opening or cavity thereof, or it may be provided in the housing and emitting/receiving the radiation through a window. If additional components are provided they may, in addition to the real purpose thereof, also protect the laser. In one situation, a light guide, such as a flexible light guide, may be provided between the laser, provided inside the system housing, and a surface thereof.

The vertical-cavity surface-emitting laser (VCSEL) usually is a type of semiconductor laser diode with laser beam emission perpendicular from the top surface, contrary to conventional edge-emitting semiconductor lasers (also in-plane lasers) which emit from surfaces formed by cleaving the individual chip out of a wafer.

The VCSEL(s) with integrated photodetector is/are positioned so as to emit radiation in a direction away from the housing and toward positions and directions in which the perfused part(s) is/are provided when the housing is provided in the ear canal. Using a VCSEL with integrated Photodetector positioned so that radiation emitted from the optical cavity may be reflected or scattered by the blood perfused part and then re-enter the optical cavity enables the use of so-called self-mixing interferometry as the detection principle.

Self-mixing interferometry (SMI) is a measurement technique, in which a laser beam is reflected from an object and fed back into the laser. The reflected light interferes with the light generated inside the laser, and this causes changes in the amplitude and frequency of the output of the laser. Information about the target object, like position and velocity can be obtained by analysing these changing properties, which are picked up by or represented in the radiation reaching the photodetector.

A blood perfused part of the ear canal may be any part of the ear canal, as all parts thereof are perfused by blood so as to be supplied by blood. Usually, blood perfusion is a transport of the blood in blood vessels of tissue, such as arteries, veins, alveoli or the like. Due to the perfusion, the blood is moving in the tissue of the part. Often, the portion of the blood perfused part receiving and emitting radiation is so small, that the blood may be said to have a single direction of movement. However, for this measurement, the blood may flow in multiple directions.

Usually, the blood perfused part is a surface part of the ear canal. This may be in the outer portions of the ear canal or in the bony portion thereof.

The housing is configured to be provided in the ear canal. In one embodiment, the housing may be completely provided inside the ear canal. A housing may be custom made to fit a particular ear canal so as to contact the ear canal over extensive portions of the housing, such as over positions at least 90% of a circumference of the housing around a longitudinal axis of the ear canal. In some situations, it is desired that the housing contacts the ear canal, such as at positions where the radiation is emitted. In other situations, the housings may be provided in the ear canal in a position where no portion of the housing touches the ear canal, at least at some points in time. Portions of the ear canal may deform when the person is chewing, and during such situations, the housing may touch the ear canal.

One or more VCSELs with integrated photodetectors are provided at or in the housing. A VCSEL with integrated photodetector may be provided inside the housing or at a wall portion thereof. Alternatively, the VCSEL with integrated photodetectoror a portion thereof, such as a radiation emitting and receiving portion thereof, may extend from the housing. Extending that portion from the housing may facilitate receiving radiation from larger angles to a general direction of emission of the laser radiation.

The VCSEL(s) with integrated photodetector is/are configured to emit radiation, as they are radiation emitters and often lasers. Receiving radiation is performed by that radiation entering the optical cavity or active region of the VCSEL with integrated photodetector. This received radiation will affect the operation of the laser, and the photo detector of the VCSEL with integrated photodetector will then determine a parameter, such as an intensity, a phase, frequency spectrum or the like, often over time, of the radiation in the optical cavity.

The controller may be based on any technology, analog or digital, such as a DSP, ASIC, FPGA, processor, software controllable or hardwired. The controller may be formed by a number of such elements which are configured to communicate with each other, wirelessly or via wires.

The controller may be completely or partly received in the housing. Alternatively, communication elements, such as cables or receivers/transmitters may be provided for transporting the output signal(s) from the VCSEL(s) with integrated photodetector to the controller.

In the housing, other elements may be provided, such as a power source or a power receiver for the controller and/or VCSEL(s) with integrated photodetector. In addition, a sound generator may be provided in the housing, where the housing is for use as a hearable, hearing aid, ear bud or the like. Naturally, all other typical elements, such as filters, amplifiers, microphones, controllers, signal receivers and the like, usual for hearables may be provided if desired.

The controller is capable of receiving output signal(s) of the VCSEL(s) with integrated photodetector, and usually of the photo detectors thereof, and to determine the parameter.

A number of parameters may be determined, as will be described below.

As mentioned, the housing need not fully engage the ear canal so that relative movement may take place between the VCSEL(s) with integrated photodetector and the blood perfused part. For a number of reasons, the housing may not be desired to itself engage the ear canal.

However, such relative movement often has an impact on optical measurements, as angles may change, reflections may change, distances may change, intensities may change due to changing angles/distances/reflections, and the like. Also, in some situations, the movement or the speed of the movement may also alter the radiation launched toward the detector, such as due to the Doppler effect. Thus, both the determination of the blood flow parameter and, as described below, a determination of a relative movement, may be affected by this relative movement. However, these may be distinguished, such as by their spectral contents and/or statistical variance over time.

In one embodiment, the controller is configured to determine the blood flow related parameter by using the differences in spectral content of the signal and/or the statistical variance over time. An important blood flow related parameter that can be derived from the stochastic part of the photodetector signal is the pulsatile behaviour of the blood flow. The movement related part of the signal may be removed from the output signal by means of frequency selective filtering.

If multiple VCSELs with integrated photodetectors are used, each VCSEL with integrated photodetector may emit radiation toward different portions of the blood perfused area and thus a relative movement may be determined for each VCSEL with integrated photodetector. The controller may be able to determine the relative movement relative to each VCSEL with integrated photodetector from the output from each VCSEL with integrated photodetector. Alternatively, the controller may perform the movement determination on information derived from all or multiple VCSELs with integrated photodetector.

In one embodiment, the controller is configured to determine the parameter by deriving a predetermined first portion of the output signal(s) and determine the parameter from the derived portion. In this situation, the controller may be configured to derive the predetermined first portion by removing a portion of the output signal(s) having a frequency below a predetermined threshold frequency.

Clearly, the desired first portion may be derived directly from the output signal(s) or it may be obtained by removing other portions of the output signal(s).

The deriving of a portion of a signal may be performed in a number of manner, such as filtering. It is especially simple if the desired signal has a particular frequency content which the undesired portion does not have, so that a frequency filtering may suffice.

An alternative, in which such effects of relative movement are reduced or completely removed is an embodiment in which the VCSEL(s) with integrated photodetector or portion(s) of the housing, from which radiation is emitted and at which radiation is received, may engage or abut the ear canal so as to create friction or the like to prevent relative movement.

In one embodiment, the system further comprises an element for estimating a movement of the housing, such as an absolute movement/acceleration or a movement vis-à-vis the blood perfused part, where the controller is configured to receive an output of the element and perform the motion compensation also on the basis of the output from the element. This has the advantage that this element may be selected and positioned/directed to be particularly sensitive in the direction of emission of the laser radiation. On the other hand, the addition of another element in or on the housing may increase the overall cost of the housing too much. Then, the controller may be configured to determine the parameter based on the output signal(s) from which a component is removed having a frequency range of the movement as determined from the output of the element. This frequency may be merely the frequency of the movement or a frequency band around this frequency and/or any overtones being frequencies being integers multiplied by the frequency of the movement.

In another one embodiment, the controller is configured to determine the parameter based on the output signal(s) from which a component is removed having a frequency range of the movement as determined from the output of the element.

In one embodiment, the controller is further configured to determine, from the output signal(s), a relative movement between the blood perfused part and the VCSEL(s) with integrated Photodetector. In this manner, the determination of also the relative movement may be made directly based on the signal output of the VCSEL(s) with integrated photodetector, so that no additional movement sensor is required. It has been found that under some circumstances, the contribution of the relative movement may be directly identified in and thus removed, from the output of the VCSEL with integrated photodetector, which makes the subsequent determination of the parameter independent of the relative movement.

Often, relative movement between the VCSEL with integrated photodetector and the tissue/blood creates movement artifacts in the output signal in the form of frequencies up to about 10kHz. A constant relative velocity would generate a single frequency, so more complex and varying movement will output varying frequencies but usually with frequencies below 10kHz, such as below 5kHz, such as below 2kHz, such as below 500Hz. Then, the parameter or a parameter may be determined from this lower frequency information.

Alternatively, such frequencies or portions may be removed or dampened, such as 3dB or more, such as 6dB or more, such as 9dB or more, from the output signal(s).

Then, blood flow generated information in the output signal may be seen as a higher frequency and more broadband noise as light scattered by blood cells will have a Doppler shift due to the velocity of the blood cells. The stochastic process of the scattering is caused by the variation of the number of blood cells in the illuminated volume, the Brownian variation in velocities (both in magnitude and direction) of the blood cells and the unpredictable number of scatter occurrences which the light encounters on its way through the tissue. Then, the information, in this signal, representing the velocity of the blood cells, will be seen as stochastic broadband noise. Often, the relevant portions of the output signals are in the interval of 50Hz-50kHz, such as 100Hz-50kHz, such as 1Khz-45kHz, such as 5-40kHz, 10-40kHz, 5-20kHz, 10-20kHz or the like. It may be decided to avoid using frequencies below 200Hz or 100Hz due to the potential contamination of the signal by motion artefacts. Alternatively, such motion artefacts may be estimated by other means, such as the above-mentioned sensor, so that also the lower frequencies in the output signal(s) may be used in the determination of the blood flow parameter. Also, an output of a movement sensor may be used for determining a filtering frequency so that frequencies exceeding a frequency detected by the movement sensor are removed before the blood flow parameter is determined from the remaining signal. In this context, movement sensors may be accelerometers, IMUs (Inertial Measurement Unit) or the like.

A level of this higher frequency noise may be taken to represent a magnitude of the velocity of the moving blood cells.

Thus, in one situation, the controller is configured to remove a portion of the output signal(s) having a frequency below a predetermined threshold frequency. Thus, a simple filtering may be performed, in some circumstances.

Filtering in the frequency domain can be obtained in many different ways, where filtering can be done on analog signals, digital signals, implemented in time-domain or in frequency domain. Selected frequency bands may be suppressed or enhanced.

Filtering can be performed using fixed filtering frequencies or programmable filter frequencies.

Filtering can also be adaptive to the signal or to the interference present in order to optimize the separation between different signal components.

Then, in a simple embodiment, a high-pass, low-pass or band-pass filter may be used, where the threshold frequency(ies) is/are set accordingly.

In a preferred embodiment, the controller is configured to determine the parameter, from the output of the VCSEL(s) with integrated photodetector, based on a Doppler shift of the radiation received vis-à-vis the radiation emitted, where the Doppler shift is caused by the flowing blood or blood flow velocity. Radiation scattered by elements, such as constituents of blood, which move will by nature impart a frequency change to the scattered radiation due to the Doppler effect. Thus, the scattered radiation will have a frequency slightly different from that received from the laser. It is noted that light may be scattered multiple times between emission from the VCSEL(s) with Integrated Photodetector and the entering back into the VCSEL(s) with Integrated Photodetector.

This Doppler shifted radiation is then re-entered into the VCSEL(s) where it affects the operation of the VCSEL. This effect may be determined in the output of the photodiode of the VCSEL(s) with Integrated Photodetector.

Different applications exist of the utilization of the Doppler effect.

Laser Doppler Velocimetry (LDV), also known as Laser Doppler Anemometry (LDA), is the technique of using the Doppler shift in a laser beam to measure the velocity in transparent or semi-transparent fluid flows or the linear or vibratory motion of opaque, reflecting surfaces. The measurement with laser Doppler anemometry is absolute and linear with velocity and requires no precalibration.

Laser Doppler Flowmetry (LDF) is a method used to assess blood flow in microvascular systems. When shining a laser beam onto perfused tissue, a part of the light is reflected at the skin surface. The remaining light will penetrate the tissue and scatter around until it is either absorbed by or re-emitted from the tissue. This manner of operating the system is especially interesting.

Thus, the radiation is selected so that it may penetrate into the blood perfused part. For this use, a wide range of wavelengths may be used, such as 850nm. Preferably, at least 1% of the radiation penetrates at least 100nm into the blood-perfused tissue in an ear canal of a person. Preferably the wavelength is tuned to the specific blood analyte(s) under test. In PPG like applications, the typical the wavelength ranges from Near infrared to UV (3um to 400 nm). The same wavelengths are quite useful in this context.

When light is scattered by a moving object, like cells in a blood flow, the scattered light will be Doppler shifted, changing its wavelength relative to the incoming light. The number of scatter events, the scatter direction and the velocity of the object influence the magnitude of the Doppler shift.

Given that light scattering, in this sense, is a stochastic process, pointing a laser at blood flow will induce broadband noise also referred to as speckle noise. When the blood flow velocity changes periodically with the heartbeat, the stochastic component contains information on biometric parameters like HR, IBI, blood pressure and more.

Thus, the processor may be configured to determine the parameter based on the theory of Doppler shifting under such circumstances. This is described further below.

As mentioned above, it may not be desired that the housing itself engages the ear canal or engages it to a degree where the housing stays in a desired position even if the user moves.

Thus, in some situations, the system further comprises a fixing element, often in the form of a so-called dome, configured to more or less fix the housing in relation to the ear canal. This element may primarily aim at fixing or limiting the housing along a longitudinal direction of the ear canal, so that the housing may move toward and away from the ear canal surface in a direction perpendicular to this. In case the system consists of multiple parts (e.g. a part in the ear canal and a part outside the ear canal) the parts can be connected mechanically and electrically. This connection may also act to fix the ear canal part in the longitudinal direction in ear canal.

Often, the VCSEL(s) with integrated photodetectors will be directed at an angle to the longitudinal direction and sometimes directly perpendicular to this direction.

A single VCSEL with integrated photodetector may be provided. Alternatively, multiple VCSELs with integrated photodetectors may be provided. It may not be desired to rely on the output of a VCSEL with integrated photodetector if it is too far from the perfused area onto which it launches radiation. Thus, it may be desired to provide multiple VCSELs with integrated photodetectors emitting radiation in different directions, such as at different angles to the longitudinal direction of the ear canal or, when projected on to a plane perpendicular to the longitudinal direction, along different directions in that plane. Thus, if the housing moves in a direction perpendicular to the longitudinal direction, one VCSEL with integrated photodetector may approach the ear canal wall where another may distance itself from its portion of the ear canal wall. Then, there may always be a VCSEL with integrated photodetector within the desired distance.

Another aspect relates to a method of operating the system according to the first aspect of the invention for determining a parameter related to blood flow of a blood perfused part of an ear canal, the method comprising:

- providing the housing configured to be provided in the ear canal,

- the one or more VCSEL(s) with integrated photodetectors emitting radiation to and receiving radiation from a surface of the ear canal and

- the controller receiving output signal(s) from the VCSEL(s) with integrated photodetector and determining the parameter related to the blood flow.

Naturally, all aspects, embodiments, situations and considerations may be freely exchanged. Thus, all considerations made in relation to the above first aspect of the invention, as well as all below aspects, are equally valid in this respect.

In one embodiment, the parameter is determined by deriving a predetermined first portion of the output signal(s) and determine the parameter from the derived portion. The controller may derive a first predetermined portion of the output signal(s) and determines the parameter from the derived portion.

In one embodiment the predetermined first portion is derived by removing a portion of the output signal(s) having a frequency below a predetermined threshold frequency.

That or another embodiment further comprises the step of estimating a movement of the housing, where the movement is taken into account in the determination of the parameter.

Then, the parameter may be based on the output signal(s) from which a component is removed having a frequency of the movement as determined from the output of the element.

That or another embodiment further comprises the step of determining, from the output signal(s), a relative movement between the blood perfused part and the VCSEL(s) with integrated Photodetector.

Then, the relative movement may be determined from a predetermined second portion of the output signal(s). The relative movement may be determined, in an adaptable manner, from the output signal(s).

An embodiment further comprises the step of fixing the housing in relation to the ear canal.

In an embodiment the parameter is determined, from the output of the VCSEL(s) with Integrated Photodetector, based on a Doppler shift of the radiation received vis-à-vis the radiation emitted, where the Doppler shift is caused by the blood flow.

Then, the controller may derive the first predetermined portion by removing a portion of the output signal(s) having a frequency below a predetermined threshold frequency.

One embodiment further comprises the step of an element estimating a movement of the housing, where the controller receives an output of the element and performs the determination of the parameter also on the basis of the output from the element.

One embodiment further comprises the step of determining, from the output signal(s), a relative movement between the VCSEL(s) with integrated Photodetector and the blood perfused part. Then, the relative movement may be determined from a predetermined second portion of the output signal(s).

In one embodiment, the determination of the relative movement from the output signal(s) is performed in an adaptable manner. It is noted that the relative movement may depend on a number of factors, such as the movements performed but also from person to person. Different persons may have different deformations of the ear canal when speaking, running, chewing or the like, so that it may be preferred to adapt the determination of the movement to the person. This may take place over time where the movement determination is first a standard determination but that the activity of the person and the relative movement is monitored so that the determination of the relative movement may be improved for the pertaining person.

As mentioned above, the movement determination and the filtering may be assisted by the use of a separate element for determining relative movement or absolute movement of the system.

As indicated above, in a preferred embodiment, the controller determines the parameter, from the output of the VCSEL(s) with integrated photodetector, based on a Doppler shift of the radiation received vis-à-vis the radiation emitted, where the Doppler shift is caused by the blood flow. In that situation, the blood or constituents thereof will scatter the radiation, where the scattered radiation will experience a Doppler shift. This may be determined from the output of the VCSEL(s) with integrated photodetector.

As indicated above, the method may further comprise fixing the housing in relation to the ear canal, such as using a so-called dome.

A third aspect of the invention relates to a system for determining a parameter related to blood flow of a blood perfused part, having a surface, of a person or animal, the system comprising:

- a housing,

- one or more VCSEL(s) each with Integrated Photodetector, each being provided at or in the housing, the VCSEL(s) with Integrated Photodetector being positioned so as to emit radiation to and receive radiation from the surface and

- a controller configured to:

  - receive the output signal(s) from the VCSEL(s) with Integrated Photodetector,

  - determine, from a first predetermined portion of the output signal(s). the parameter related to the blood flow, and

- determine, from a second predetermined portion of the output signal(s), a relative movement between the blood perfused part and the VCSEL(s) with Integrated Photodetector. As mentioned above, all embodiments, situations and considerations made above and below are equally relevant in relation to this aspect of the invention.

Thus, the VCSEL(s) with integrated photodetector and controller may be as described above. However, the housing no longer is required to fit inside an ear canal of a person. Thus, the housing may be larger and may comprise additional elements, such as user interfaces, other sensors, sound generators, microphones, or the like. The present housing may be configured to be attached to or fixed to the human body, such as a skin portion thereof. The housing may form part of a watch, ring, pendant, or the like, and fixing elements may be provided for attaching the housing to the human body in the desired position, such as on an arm, hand, wrist, leg, foot, neck, torso, face or the like.

The determination of the parameter relating to the blood flow may be as described above.

The movement determination based on a portion of the output signal(s) is described further above. Thus, the controller may be configured to derive the first predetermined portion by removing a portion of the output signal(s) having a frequency below a predetermined threshold frequency.

Also, as described above, the system may further comprise an element for estimating a movement of the housing (vis-à-vis the blood perfused part), where the controller is configured to receive an output of the element. Then, the controller may take this into account when determining the parameter. Additionally, or alternatively, the controller may use the output of the element in the determination of the relative movement.

It may be assumed that the surface of the skin is at rest, so that the measured absolute movement of the system may be used as estimate for the movement of the system relative to the skin. Alternatively, the measured absolute movement may be taken into account in the determination of the relative movement.

As indicated above, the blood perfused part may be part of an ear canal of the person or animal, and wherein the housing is configured to be provided in the ear canal.

In one embodiment, the system further comprises a fixing element configured to fix the housing relative to the blood perfused part. When the perfused part forms part of an ear canal, this fixing element may be a so-called dome.

As indicated above, the controller is preferably configured to determine the parameter, from the output of the VCSEL(s) with integrated photodetector, based on a Doppler shift of the radiation received vis-à-vis the radiation emitted, where the Doppler shift is caused by the blood flow.

A fourth aspect of the invention relates to a method of operating the system according to the third aspect of the invention for determining a parameter related to blood flow of a blood perfused part of a person or animal, the method comprising:

- providing the housing,

- the one or more VCSELs each with Integrated Photodetector emitting radiation to and receiving radiation from the surface and

- the controller:

  - receiving output signal(s) from the VCSEL(s) with Integrated Photodetector,

  - deriving a first predetermined portion of the output signal(s) and determining, based thereon, the parameter, and

  - deriving a second predetermined portion of the output signal(s) and determining, based thereon, a relative movement between the blood perfused part and the VCSEL(s) with Integrated Photodetector.

As indicated above, the blood flow related parameter and the relative movement may be determined from the output signal(s), such as by using a portion having a frequency above a threshold frequency. Then, the first predetermined part may be obtained by removing a portion having a frequency below the threshold frequency.

In one embodiment, the method further comprises the step of an element estimating a movement of the housing (vis-à-vis the blood perfused part), where the controller receives an output of the element and performs the determination of the parameter and/or the relative movement on the basis of the output from the element. As described above, this element may output a signal useful in any of the situations.

As mentioned, the blood perfused part preferably is part of an ear canal of the person or animal, and wherein the housing is provided in the ear canal.

It may be desired that the method further comprises fixing the housing relative to the blood perfused part, such as an ear canal.

In one embodiment, the controller determines the parameter, from the output of the VCSEL(s) with integrated photodetector, based on a Doppler shift of the radiation received vis-à-vis the radiation emitted, where the Doppler shift is caused by the blood flow.

A fifth aspect of the invention relates to a system for determining a parameter related to blood flow of a blood perfused part of a person or animal, the system comprising:

- a housing,

- one or more VCSELs, each with Integrated Photodetector, each being provided at or in the housing, the VCSEL(s) with Integrated Photodetector being positioned so as to emit radiation to and receive radiation from the blood perfused part and

- a controller for receiving output signal(s) from the VCSEL(s) with Integrated Photodetector and determine the parameter, from the output of the VCSEL(s) with Integrated Photodetector, based on a Doppler shift of the radiation received vis-à-vis the radiation emitted, where the Doppler shift is caused by the blood flow.

As described, all above and below embodiments, considerations, situations and the like are equally relevant in this connection.

The housing may be as described in relation to the third or first aspects and the VCSEL(s) with integrated photodetector and controller may be as described above.

When the VCSEL(s) with integrated photodetector are used together with the Doppler shifted radiation, the output of the VCSEL(s) with integrated photodetector directly relates to the frequency shift as the operation of the VCSEL(s) with integrated photodetector will be affected by the Doppler shifted radiation.

The determination of the parameter from the output signal(s) may be as described above. Thus, a first portion may be determined and obtained, such as by removing a portion of the output signal(s), so that the first portion is obtained and is suitable for the determination of the parameter.

In one embodiment, the system further comprises an element for estimating a movement of the housing (vis-à-vis the blood perfused part), where the controller is configured to receive an output of the element and determine the parameter also on the basis of the output from the element.

Preferably, the blood perfused part is part of an ear canal of the person or animal, and wherein the housing is configured to be provided in the ear canal. Alternatively, a number of other positions in and on the human body are described above.

It may be desired to provide a fixing element configured to fix the housing relative to the blood perfused part, such as in an ear canal of a person. Fixing is described above.

A sixth aspect of the invention relates to a method of controlling a system according to the fifth aspect of the invention for determining a parameter related to blood flow of a blood perfused part of a person or animal, the method comprising:

- providing the housing,

- the one or more VCSELs each with Integrated Photodetector emitting radiation to and receiving radiation from the blood perfused part and

- the controller receiving output signal(s) from the VCSEL(s) with Integrated Photodetector and determining the parameter, from the output signal(s) and based on a Doppler shift of the radiation received vis-à-vis the radiation emitted, where the Doppler shift is caused by the blood flow.

The determination of the parameter from the output signal(s) is described further above. Thus, the controller may determine the parameter based on a derived first predetermined portion of the output signal(s). The deriving of the first predetermined portion may comprise removing a portion of the output signal(s). The removing step comprises removing a portion of the output signal(s) having a frequency below a predetermined threshold frequency.

In one embodiment, the method further comprises an element estimating a movement of the housing (vis-à-vis the blood perfused part), where the controller receives an output of the element and determines the parameter also on the basis of the output from the element. Alternatively, the movement may be determined, if at all, from the output of the VCSEL(s) with integrated photodetector.

Preferably, the blood perfused part is part of an ear canal of the person or animal, and wherein the housing is provided in the ear canal.

As described, it may be desired to further comprise fixing the housing relative to the blood perfused part using a fixing element.

In general, it is desired that the VCSEL(s) with integrated photodetector is/are configured to output radiation capable of travelling into the blood perfused part. The wavelength, or a wavelength, may be selected for or tuned to a particular blood analyte under test. Often, wavelengths used also for PPG, are in the range from NIR to UV - $3\mu$m-400nm. Thus, the VCSEL(s) with integrated photodetector preferably output radiation travelling into the blood perfused part.

A number of different parameters may be determined. In one embodiment, the parameter is a velocity of blood in the blood perfused part. In other embodiments, the parameter may beSP02, particle sizes, particle concentration, blood pressure, breathing rate, perfusion, pulse, heart rate variability, heartrate, or the like.

The movement need not be determined to be compensated for. As described, a simple filtering may remove the contribution of the movement. From the information filtered away, the movement may be determined. Thus, in one embodiment, the controller is configured to also determine relative motion between the blood perfused part and the VCSEL(s) with integrated photodetector. This information may be used for identifying portions of the output of the VCSEL(s) with integrated photodetector for determining other parameters of the blood flow. Alternatively, the information may be used generally as activity information from activity trackers such as watches, phones and the like. Thus, the method may further comprise the step of determining a relative motion between the blood perfused part and the VCSEL(s) with integrated photodetector.

As mentioned above, it may be desired that fixing elements are provided for fixing the housing in relation to the blood perfused part. It may be desired that the fixing element maintains at least substantially a desired non-zero distance between the blood perfused part and an outer surface of the housing and/or VCSEL with integrated photodetector, so that relative motion between the body part and the system is allowed, which will provide information as to an activity of the person. Also, no contact will decrease any irritation from wearing the system.

On the other hand, providing contact between the body part and the system may reduce any movement artifacts from the system and the output signal(s) and may provide a better coupling of light to/from the tissue.

In the following, preferred embodiments of the invention will be described with reference to the drawing, wherein:

- Figure 1 illustrates a first embodiment of the invention,

- Figure 2 illustrates primary elements of a VCSEL with integrated photodetector,

- Figure 3 illustrates the use of a VCSEL with integrated photodetectorand the Doppler effect for determining blood flow,

- Figure 4 illustrates components of the VCSEL with integrated photodetector output signal,
- Figure 5 illustrates the use of a VCSEL with integrated photodetector for illuminating a volume,
- Figure 6 illustrates an embodiment of the invention in the form of a hearable,
- Figure 7 illustrates a jogger comprising different embodiments of the invention,
- Figure 8 illustrates an embodiment of the invention in the form of a patch,
- Figure 9 illustrates an embodiment of the invention in the form of a ring
- Figure 10 illustrates a person wearing different embodiments of the invention,
- Figure 11 illustrates a system architecture of the invention in the form of a hearing instrument,
- Figure 12 illustrates a system architecture of the invention in the form of a body worn device and
- Figure 13 illustrates a data flow diagram of the invention.

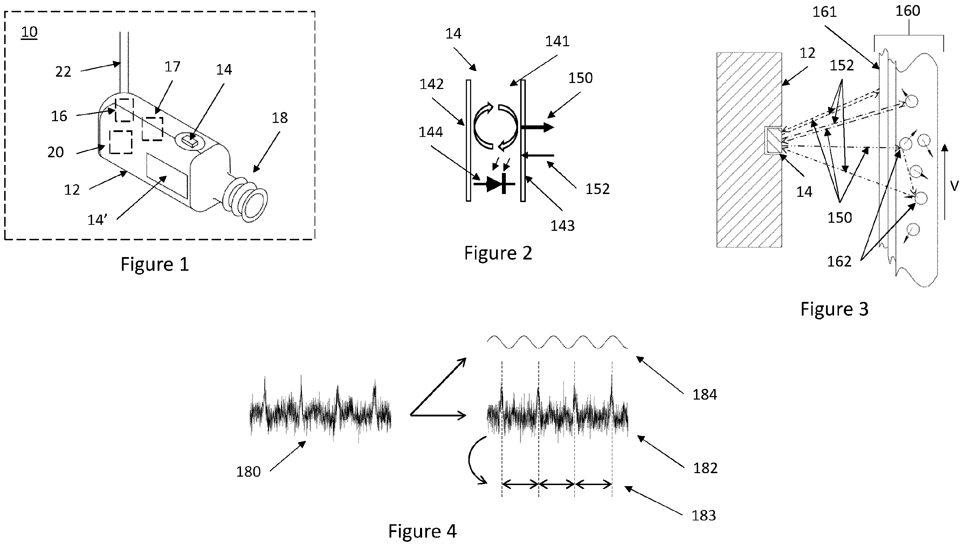

In figure 1, a system 10, in the form of a hearable, 10 is illustrated having a housing 12 configured to be provided in an ear canal of a person. One or more VCSELs with integrated photodetectors 14 are provided on or in the housing so as to be able to emit radiation in a direction away from the housing and to receive radiation travelling in the opposite direction. The VCSELS with integrated photodetector may be positioned outside of the housing or behind windows 14'. In the housing, a sound generator 16, often called a receiver, may be provided for outputting sound from a spout 18, usually in the direction of the ear drum of the ear canal.

Further, a processor 20 is provided, often in the housing 12, for receiving output signals from the VCSEL(s) with integrated photodetector and for outputting a parameter or information from which the parameter may be determined. The processor 20 may be configured to communicate with other electronic components via a connector, communication port or cable 22, which may also carry power and/or other signals for the housing 12, sound generator 16 or the like.

In the present context, cf. figure 2, a VCSEL with Integrated Photodetector 14 could be a Vertical Cavity Surface Emitting Laser with Integrated Photodetector 144. A vertical-cavity surface-emitting laser (VCSEL) is a type of semiconductor laser diode with laser beam emission perpendicular from the top surface, contrary to conventional edge-emitting semiconductor lasers (also in-plane lasers) which emit from surfaces formed by cleaving the individual chip out of a wafer. We note that the term VCSEL includes Vertical external cavity surface emitting lasers (VECSEL).

Clearly, the sensor or detector 144 could be positioned suitably in relation to the VCSEL, but when it is monolithically integrated with the VCSEL, a very compact solution is obtained which may be particularly relevant when space is an issue, such as when the hearable is for use in an ear canal of a person.

A VCSEL with integrated photodetector and a manner of determining a parameter relating to blood flow of a blood perfused portion of a person, such as in an ear canal of the person, is seen in figure 3.

In figure 2, a VCSEL with integrated photodetector 14 is seen having an active region or optical cavity 141 provided between two reflecting portions 142 and 143, often formed by Bragg reflectors. Radiation 150 is emitted from the optical cavity 141 through the reflecting portion 143, which is not completely reflecting. Radiation 152 received into the cavity 141 will interfere with the operation of the VCSEL in a manner which will be detectable by the detector 144, cf below.

In figure 3, the radiation 150 is launched on to the skin 161 of a tissue portion 160, such as an ear canal, of the person. Inside the tissue, such as the ear canal, blood is flowing. In blood, different constituents exist, such as red blood cells 162. The flood flows in arteries, veins, capillaries and at an average velocity V (averaged over the exposed volume), which may vary over time in amplitude and direction, and blood cells have individual velocity vectors which are indicated by arrows. Clearly, the velocity of the flowing blood will depend on the heartbeat of the person. Also, the stiffness of the blood vessels will affect the velocity, as the velocity will be higher in stiffer blood vessels not able to expand to compensate for an increasing blood pressure due to the heart pumping.

In the present illustration, the blood flows at velocity V in an upward direction.

Part of the emitted radiation 150 will be reflected by the skin surface and revert toward the element 143 to re-enter the optical cavity 141.

As the radiation 150 is capable of entering the tissue of the person, and thus penetrating the skin or other surface 161, part of the emitted radiation will penetrate the skin and be scattered by the blood and in particular the particles or cells thereof. This scattering will both redirect the radiation but will also impart, due to the Doppler effect, a wavelength shift thereof. Some of this radiation, radiation 152, is scattered back toward the optical cavity 141.

As the optical cavity 141 forms part of the laser of the VCSEL with integrated photodetector, the radiation 152 entering the optical cavity 141 will affect the radiation generation of the laser and will affect both the amplitude and the frequency of the radiation emitted and thus in the optical cavity.

The detector 144 is positioned so as to determine the power of the radiation in the optical cavity 141. Thus, the frequency and amplitude of this power is affected by the radiation reflected from the blood cells 162 and thus the velocity thereof.

Thus, from the signal output of the detector 144, this velocity may be determined.

The spectral power density S(v) of the photo current fluctuations of the output signal(s) of the detector 144 may be determined from the output signal during a number of time intervals, such as every 10ms. Then, the first moment M1 may be determined for each point in time:

$$M_1(t) = \int_{v1}^{v2} v \cdot S(v,t)\, dv$$

Where v is the frequency, a=lkHz, for example, and b=20kHz, for example. The upper and lower frequency limits can be varied in order to achieve optimal separation between the movement portion of the signal and the blood flow velocity portion.

This M1 will be a measure of the blood flow velocity. Then, the moments M1 may be plotted against time to illustrate the variation over time of this velocity. From this, the heart rate, for example, may be determined.

Alternatively, the 0$^{th}$ moment may be used for determining the parameter.

Also, the use of an analog circuit may be chosen to bandpass filter and integrate over time, in order to arrive at the blood flow.

Yet another manner would be to use a number of M1 bands and pick the best one for the determination.

It could be preferred to calculate the spectral power density at pre-defined points in time from the signal acquired over a defined time interval preceding the present point in time, which could be selected to be the interval between two sequential points in time.

The k-th moment of the spectral power density is:

$$M_k(t) = \int_{v1}^{v2} v^k \cdot S(v,t)\, dv$$

It is noted that the system measures the velocity of the blood cells moving in the tissue, and not necessarily the blood flow. The velocity is the distance which an object (solid, liquid or gas) moves with respect to time (i.e., the distance travelled per unit of time). In the case of blood flowing in a vessel, the velocity is often expressed in the units of cm/sec. In contrast, flow is the volume of a liquid or gas that is moving per unit of time. For blood flowing in a large vessel, flow is often expressed in the units of ml/min (cm$^3$/min; 1 ml = 1cm$^3$).

In some situations, the VCSEL with integrated photodetector 14 is allowed to move relative to the tissue or portion 160 . Naturally, this movement will in itself give rise to a Doppler shift and amplitude modulation of the radiation 152. This Doppler shift is seen both in the radiation reflected from the surface of the skin 161, from scattering from within the tissue 160 as well as from the radiation scattered by the blood 162.

Thus, the photodetector output signal will be a superposition of the artifacts caused by movement of the tissue 160 relative to the VCSEL with integrated photodetector 14 and that caused by the movement of the blood in the tissue 160. Figure 4 illustrates the components of the photodetector output signal 180 and the later splitting up thereof into the signal 182, being a high frequency portion, relating to the Doppler shift and amplitude modulation caused by the velocity of the blood 162 and the signal 184, being a low frequency portion, relating to the amplitude modulation caused by the relative movement between the VCSEL with integrated photodetector and the tissue. This relative movement may be caused by the user moving, such as running, causing the housing 12 to move inside the ear canal. Alternatively or additionally, the tissue 160 may swell periodically due to the blood pressure variations caused by the heart beating.

However, it has been realized that the changing distance between VCSEL with integrated photodetector and reflective tissue, will be present as deterministic discrete frequencies, whereas the biometric information relating to the blood flow is present in the photodetector output in the form of broadband stochastic noise. The bandwidth of the noise can be for example between 1kHz and 50kHz.

Thus, in this embodiment, the output signal is processed into biometric signals independent of movement artifacts. This processing can be performed in several ways. In one situation, the biometric signals are in a higher frequency band than the movement artifacts. Then, the average of the power spectral density of the noise between e.g. 10-50kHz may be representative of the blood flow velocity without movement artifacts.

A simple method would be to apply a low pass filter to the photodetector output signal which removes the stochastic broadband noise.

In fact, even if movement artifacts will appear at higher frequencies, their deterministic nature makes them distinguishable from the stochastic broadband noise. These artifacts can be removed by the signal processing by using distinguishable features such as amplitude, spectral density, and time dependency.

The motion component, 184, in the photodetector signal can be used for motion compensation of the biometric signals. Using the motion component of the photodetector signal as input for motion compensation comes with the benefit that it is measured in the same domain as the biometric signal. It is using the same optical signal and as such is subject to the same optical disturbance.

Alternatively, a separate movement sensor 17, such as an IMU, could be used for estimating the movement for the movement compensation to be based thereof. However, using a separate movement sensor would entail measuring in a different domain, e.g. light versus inertia. As there is not always a high correlation between the two domains, inertial movements do not always create optical disturbances and optical disturbances are not always caused by inertial movements. As such, intrinsic compensating is better than extrinsic compensating.

Thus, the photodetector signal may be adapted before or during the determination of the blood flow parameter, such as a velocity of the blood flow or blood cells. Other parameters may be determined based on the photodetector output signal, such as a perfusion of the tissue, heart rate, heart rate variability, artery stiffness, blood pressure, breathing rate or the like. In figure 4, a peak detection is performed on the high frequency signal 182 (alternatively, it could be used on the signal 180) to identify heart beats and thus e.g. pulse or heart rate.

These parameters can be used to determine conditions of the system's user, such as health related conditions as atrial fibrillation or the like, his/her stress level, fitness level or the like. Clearly, the blood velocity for the same person with the same pulse will increase with increased arterial stiffness.

In addition to, or alternatively to, using the motion component for compensation, the motion features might also be used for tracking motion related metrics of the person, like e.g. cadence or activity tracking, as it is known from a plethora of trackers, such as activity watches.

Heartrate induced tissue movement is also present in the tissue. Under sedentary conditions this movement is visible in the low frequency content of the photodetector signal. The heartrate induced tissue movement can be used as a redundancy check or even as a fundamental signal for metrics like e.g. heartrate.

It is noted, referring to figure 4, that the heartrate movement signal is, as the biometric signal in the noise, caused by the pulsating of the heart. Therefore, the signals will have the same period. This fact can be used to distinguish body movement artifacts, as seen in the signal 184, from heart beat induced motion artifacts with the frequency seen at 183. Actually, a signal (not illustrated) could be derived from the signal 180 with the frequency of the peaks.

Due to the fact that the biometric derived from the noise signal 182 is a measure for the blood flow velocity, and that the signal, derivable with the frequency 183, is a measure for the amount of blood, the two signals will have different shapes. This difference will depend other tissue parameters, such as artery stiffness, blood pressure, and the like, which then also could be determined and potentially monitored over time.

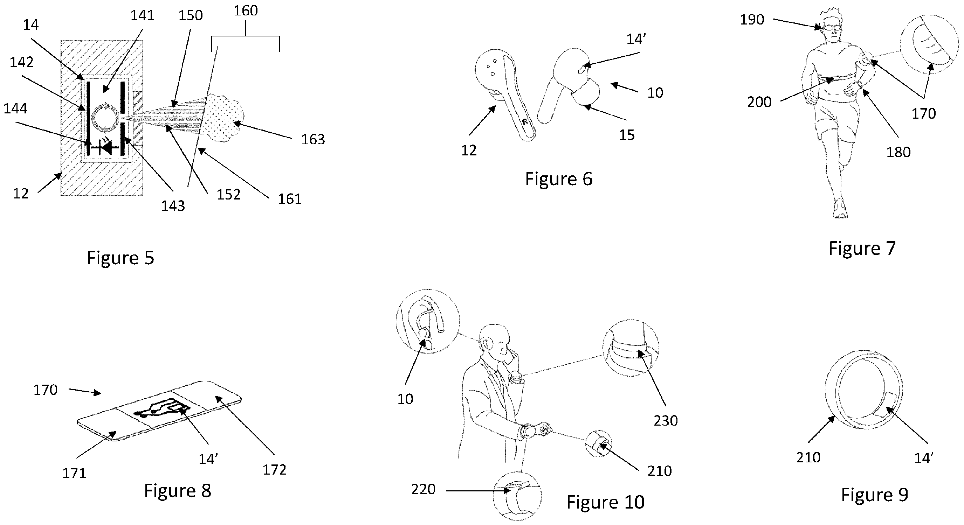

In this context, it is then noted that the output of the photodetector may be used for generating information on amplitude variation caused by relative movement between the VCSEL with integrated photodetector and the tissue or element in question. It is noted that the tissue may move with the blood pressure pulses. Then, this movement will have a, relatively small, part of pulsatile information, which may be used for determining e.g. heart beat.Figure 5 is an alternative illustration to figure 3 where again the system 10 with the VCSEL with integrated photodetector is seen with the radiation 150 launched through the surface 161 and into the portion 160 where the illuminated volume is 163 from which the radiation 152 is emitted.

In general, it is noted that a VCSEL with integrated photodetector may, especially when used rather close to the tissue in question, be used without further optics, which allows the optical sensor to remain compact, such as for use in small housings and volumes. Alternatively, other optical components, such as lenses, slits, reflectors or the like, may be provided outside of the VCSEL for protection thereof and/or beam forming and the like.

The assembly 10 may be a hearable, such as an ear bud or a hearing aid, as seen in figure 6, further comprising an element, such as a so-called dome 15, for fixing or attaching the housing 12 to the tissue, such as the ear canal. In this manner, the relative movement between the VCSEL with integrated photodetector 14, emitting radiation out through the window 14', and the tissue 160 may be limited and/or the housing 12 may remain in place during the person's use or activity.

Other types of fixing elements may be pads, extensions or the like of the housing 12.

The fixing element may allow a distance from the housing 12 or VCSEL with integrated photodetector 14 to the tissue 160, or it may have the housing or VCSEL with integrated photodetector and tissue engage so that there is no distance. Maintaining no distance may simplify the set-up and the calculations as some of the relative movements are then avoided. Allowing a distance between the housing and the ear canal allows the ear canal to move, such as during chewing or running, without the housing engaging the ear canal and exerting forces on the ear canal.

Also, the housing may be fixed in relation to any other portion of the person, such as any portion of the person's skin, such as if forming part of a watch, ring, head band, arm band, clothing, when fixed using a resilient fixture, such as a waist band, arm band or the like.

Figure 7 illustrates a jogger wearing different embodiments of the invention, such as in the form of a patch 170, a watch 180, glasses 190 or a chest band 200, each comprising a system 10. Figure 8 illustrates the patch 170 having two adhesive portions 171 and 172 and a central portion comprising a window 14' behind which the VCSEL with integrated photodetector 14 is provided transmitting radiation through the window 14'.

Figure 9 illustrates a ring 210 having a window 14' behind which the VCSEL with integrated photodetector 14 is provided so as to launch radiation out through the window 14'. Similar embodiments would be seen in a watch 180, an arm band, the chest band, and the like.

Figure 10 illustrates a person wearing a number of embodiments of the invention, including a ring 210, a watch 220, a wrist band 230 and a hearable 10.

When the housing is not to be provided in the narrow ear canal of the person, the housing may be made larger and more VCSELs with integrated photodetectors may be used or even VCSELs where the photo detector is not integral with the VCSEL.

In the situation with the use in the ear canal, hearables may be split up into portions, where one portion is for positioning in the ear canal and another portion is positioned outside of the ear canal, such as behind the ear or ear lobe of the person and/or a portion may be included in another electronic device of the user, such as a watch, phone, computer, tablet or the like.

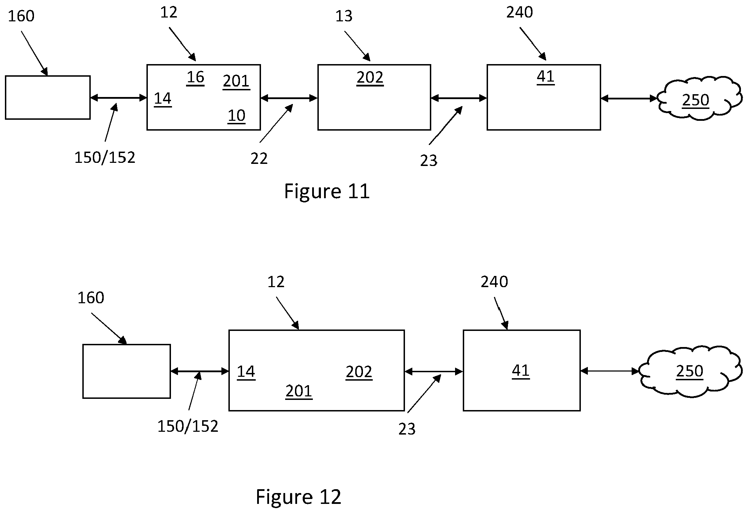

In figure 11, relevant portions of the assembly are represented together with possible manners of dividing these up into different parts of an assembly. In figure 11, the tissue portion 160 is seen to the left, where a first housing 12 comprises the sound generator 16 together with the VCSEL with integrated photodetector 14. A processor 20 is often provided for the calculations and signal handling. In this embodiment, this processor has been subdivided into relevant portions: front end electronics 201, such as comprising an A/D converter or an amplifier, provided in the housing 12 for positioning in an ear canal of the user, and a further processor 202 provided in another housing, 13, such as for providing behind the ear of the person. Data may be exchanged between the housings 12 and 13 via the cable 22. The further processor 202 may perform additional signal processing, such as digital signal processing, to arrive at the metrics described above.

The housing 12 may comprise multiple VCSELs with integrated photodetector if desired, analog front-end electronics, A/D converter for example. Also, the housing 12 may comprise a receiver if used as a hearing aid or hearable. A dome may be provided for acoustical sealing and fixation in the ear canal.

The other housing 13 may comprise a battery for powering the elements in the housings 12 and 13, a digital signal processor, memory and/or communication interface, such as Bluetooth interface, if desired.

The filtering described above may be performed in any of the processors 201 and 202. A further element 240 may be provided for receiving the output of the processor 202 for further processing, displaying of information to the user, or for the user to affect the operation of the system. A user interface 41 may be provided for interaction with the user. The further element 240 may communicate wirelessly (or via wires), such as via Bluetooth 23, with the housing 13 and/or the further processor 202. The further element 240 may be a pc, laptop, mobile phone, pad or the like and may run one or more apps, programs or the like for communicating with the processor 202, deriving parameters or metrics based on the data from the VCSEL with integrated photodetector 14 and/or interact with the user via the user interface 41.

Naturally, the further element 240 may communicate with cloud-based servers or services 250 for additional data handling, receiving algorithms for the data processing, or the like.

In this embodiment, the first housing 12 may comprise the VCSEL with integrated photodetector 14, the sound generator 16, analog electronics for handling signals for the sound generator 16 and/or signals from the VCSEL with integrated photodetector 14, as well as any desired fastening elements, such as a dome.

The other housing 13 may comprise a communication interface toward the further element 240, a signal processor 202, such as a digital signal processor, a battery for operation of the elements of the housings 12 and 13, memory for the processor and/or the like.

The further element 240 may comprise its own processor, memory for that processor, software, such as apps, operating system, user interface, software development kit and the like, a display as well as desired communication interfaces, such as Bluetooth, WiFi, NFC, GSM and the like.

Another embodiment is seen in Figure 12, which is more directed at body-worn embodiments as seen in figures 7-10. In this embodiment, the portion 160 is seen to the left of a housing 12, which is body-worn and may be a ring, watch, wrist band, chest band, or the like, comprising the VCSEL with integrated photodetector 14 as well as one or more controllers performing the abovementioned functions of the processors 201 and 202. This housing 12 may then communicate with the further element 240 as described above.

Naturally, the cloud-based services and servers 250 may be included in the embodiment of Figure 12 if desired.

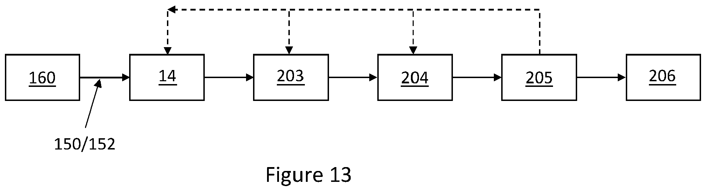

In Figure 13, a signal and data flow diagram is illustrated in which the blood perfused portion 160 receives radiation from and delivers radiation to a signal acquisition element comprising the VCSEL with integrated photodetector 14 feeding a signal back to a signal conditioning element 203 feeding a signal back to a signal separation element 204, which may comprise the abovementioned filters or other elements for performing the signal separation, and feeding a signal to a parameter extraction unit 205 again outputting a signal to a parameter output and/or presentation unit 206, which may be a computer, mobile phone, laptop or the like.

Clearly, the signal acquisition element may comprise one or more VCSEL(s) with detectors and the signal conditioning element may comprise analog gain an filtering and/or analog to digital conversion. The signal separation element may comprise multiple band filtering, such as low pass filtering for tissue motion determination, high frequency noise filtering for blood velocity determination, low frequency and high frequency filtering for system performance determination. Also parameters relating to motion and velocity may be used for generating motion corrected velocity determination.

The parameter extraction unit may derive parameters relating to e.g. blood velocity, blood volume, blood pressure, oxygen saturation, tissue movement, heart rate, heart rate variability, user activity detection or the like.

The parameter output and/or presentation unit may comprise a data storage and may output data or parameters directly to a user, a health care professional. The output may be used in or based on hearing instrument audio algorithms when the system is used in relation to a hearing instrument.

In this figure, hatched arrows indicate that a feedback may be provided for adaptive processing. Naturally, the parameters output may be compared to parameters obtained in other manners and a feedback may be provided to adapt the signal chain in order to arrive at parameters or a signal treatment which is more suitable or correct. This feedback may be used for adapting settings for signal quality optimization, for example.

EMBODIMENTS

1. A system for determining a parameter related to blood flow of a blood perfused part of an ear canal, the system comprising:

- a housing configured to be provided in the ear canal,

- one or more VCSEL(s) each with Integrated Photodetector and each being provided at or in the housing, the VCSEL(s) with Integrated Photodetector being positioned so as to emit radiation to and receive radiation from a surface of the ear canal and

- a controller for receiving output signal(s) from the VCSEL(s) with Integrated Photodetector and determine the parameter related to the blood flow.

2. A system according to embodiment 1, wherein the controller is configured to determine the parameter by deriving a predetermined first portion of the output signal(s) and determine the parameter from the derived portion.

3. A system according to embodiment 2, wherein the controller is configured to derive the predetermined first portion by removing a portion of the output signal(s) having a frequency below a predetermined threshold frequency.

4. A system according to any of the preceding embodiments, further comprising an element for estimating a movement of the housing, where the controller is configured to receive an output of the element and take this into account when determining the parameter.

5. A system according to embodiment 4, wherein the controller is configured to determine the parameter based on the output signal(s) from which a component is removed having a frequency of the movement as determined from the output of the element.

6. A system according to any of the preceding embodiments, wherein the controller is further configured to determine, from the output signal(s), a relative movement between the blood perfused part and the VCSEL(s) with integrated Photodetector.

7. A system according to embodiment 6, wherein the controller is configured to determine the relative movement from a predetermined second portion of the output signal(s).

8. A system according to embodiment 6, wherein the controller is configured to, in an adaptable manner, determine the relative movement from the output signal(s).

9. A system according to any of the preceding embodiments, further comprising a fixing element configured to fix the housing in relation to the ear canal.

10. A system according to any of the preceding embodiments, wherein the controller is configured to determine the parameter, from the output of the VCSEL(s) with Integrated Photodetector, based on a Doppler shift of the radiation received vis-à-vis the radiation emitted, where the Doppler shift is caused by the blood flow.

11. A method of operating the system according to any of the preceding embodiments for determining a parameter related to blood flow of a blood perfused part of an ear canal, the method comprising:

- providing the housing configured to be provided in the ear canal,

- the one or more VCSEL(s) each with Integrated Photodetector emitting radiation to and receiving radiation from a surface of the ear canal and

- the controller receiving output signal(s) from the VCSEL(s) with Integrated Photodetector and determining the parameter related to the blood flow.

12. A method according to embodiment 11, wherein the parameter is determined by deriving a predetermined first portion of the output signal(s) and determine the parameter from the derived portion.

13. A method according to embodiment 12, wherein the predetermined first portion is derived by removing a portion of the output signal(s) having a frequency below a predetermined threshold frequency.

14. A method according to any of embodiments 11-13, further comprising the step of estimating a movement of the housing, where the movement is taken into account in the determination of the parameter.

15. A method according to embodiment 14, wherein the parameter is based on the output signal(s) from which a component is removed having a frequency of the movement as determined from the output of the element.

16. A method according to any of embodiments 11-15, further comprising the step of determining, from the output signal(s), a relative movement between the blood perfused part and the VCSEL(s) with integrated Photodetector.

17. A method according to embodiment 16, wherein the relative movement is determined from a predetermined second portion of the output signal(s).

18. A method according to embodiment 16, wherein the relative movement is determined, in an adaptable manner, from the output signal(s).

19. A method according to any of embodiments 11-18, further comprising the step of fixing the housing in relation to the ear canal.

20. A method according to any of embodiments 11-19, wherein the parameter is determined, from the output of the VCSEL(s) with Integrated Photodetector, based on a Doppler shift of the radiation received vis-à-vis the radiation emitted, where the Doppler shift is caused by the blood flow.

21. A system for determining a parameter related to blood flow of a blood perfused part, having a surface, of a person or animal, the system comprising:

- a housing,

- one or more VCSEL(s) each with Integrated Photodetector, each being provided at or in the housing, the VCSEL(s) with Integrated Photodetector being positioned so as to emit radiation to and receive radiation from the surface and

- a controller configured to:

    - receive the output signal(s) from the VCSEL(s) with Integrated Photodetector,

    - determine, from a first predetermined portion of the output signal(s). the parameter related to the blood flow, and

    - determine, from a second predetermined portion of the output signal(s), a relative movement between the blood perfused part and the VCSEL(s) with Integrated Photodetector.

22. A system according to embodiment 21, wherein the controller is configured to derive the first predetermined portion by removing a portion of the output signal(s) having a frequency below a predetermined threshold frequency.

23. A system according to any of embodiments 21-22, further comprising an element for estimating a movement of the housing, where the controller is configured to receive an output of the element and take this into account when determining the parameter.

24. A system according to any of embodiments 21-23, wherein the blood perfused part is part of an ear canal of the person or animal, and wherein the housing is configured to be provided in the ear canal.

25. A system according to any of embodiments 21-24, further comprising a fixing element configured to fix the housing relative to the blood perfused part.

26. A system according to any of embodiments 21-25, wherein the controller is configured to determine the parameter, from the output of the VCSEL(s) with Integrated Photodetector, based on a Doppler shift of the radiation received vis-à-vis the radiation emitted, where the Doppler shift is caused by the blood flow.

27. A method of operating the system according to any of embodiments 21-26 for determining a parameter related to blood flow of a blood perfused part of a person or animal, the method comprising:

- providing the housing,

- the one or more VCSELs each with Integrated Photodetector emitting radiation to and receiving radiation from the surface and

- the controller:

    - receiving output signal(s) from the VCSEL(s) with Integrated Photodetector,

    - deriving a first predetermined portion of the output signal(s) and determining, based thereon, the parameter, and

    - deriving a second predetermined portion of the output signal(s) and determining, based thereon, a relative movement between the blood perfused part and the VCSEL(s) with Integrated Photodetector.

28. A method according to embodiment 27, wherein the controller derives the first, predetermined part by removing, from the output signal(s), a portion having a frequency below a predetermined threshold frequency.

29. A method according to any of embodiments 27 and 28, further comprising the step of an element estimating a movement of the housing, where the controller receives an output of the element and performs the determination of the parameter also on the basis of the output from the element.

30. A method according to any of embodiments 27-29, wherein the blood perfused part is part of an ear canal of the person or animal, and wherein the housing is provided in the ear canal.

31. A method according to any of embodiments 27-30, further comprising fixing the housing relative to the blood perfused part.

32. A method according to any of embodiments 27-31, wherein the controller determines the parameter, from the output of the VCSEL(s) with Integrated Photodetector, based on a Doppler shift of the radiation received vis-à-vis the radiation emitted, where the Doppler shift is caused by the blood flow.

33. A system for determining a parameter related to blood flow of a blood perfused part of a person or animal, the system comprising:

- a housing,

- one or more VCSELs each with Integrated Photodetector each being provided at or in the housing, the VCSEL(s) with Integrated Photodetector being positioned so as to emit radiation to and receive radiation from the blood perfused part and

- a controller for receiving output signal(s) from the VCSEL(s) with Integrated Photodetector and determine the parameter, from the output of the VCSEL(s) with Integrated Photodetector, based on a Doppler shift of the radiation received vis-à-vis the radiation emitted, where the Doppler shift is caused by the blood flow.

34. A system according to embodiment 33, wherein the controller is configured to derive a first predetermined portion of the output signal(s) and determine, based thereon, the parameter.

35. A system according to embodiment 34, wherein the controller is configured to derive the first predetermined portion by removing a portion of the output signal(s) having a frequency below a predetermined threshold frequency.

36. A system according to any of embodiments 33-35, further comprising an element for estimating a movement of the housing, where the controller is configured to receive an output of the element and determine the parameter also on the basis of the output from the element.

37. A system according to any of embodiments 33-36, wherein the blood perfused part is part of an ear canal of the person or animal, and wherein the housing is configured to be provided in the ear canal.

38. A system according to any of embodiments 33-37, further comprising a fixing element configured to fix the housing relative to the blood perfused part.

39. A method of controlling a system according to any of embodiments 33-38 for determining a parameter related to blood flow of a blood perfused part of a person or animal, the method comprising:

- providing the housing,

- the one or more VCSELs each with Integrated Photodetector emitting radiation to and receiving radiation from the blood perfused part and

- the controller receiving output signal(s) from the VCSEL(s) with Integrated Photodetector and determining the parameter, from the output signal(s) and based on a Doppler shift of the radiation received vis-à-vis the radiation emitted, where the Doppler shift is caused by the blood flow.

40. A method according to embodiment 39, wherein the controller determines the parameter based on a derived first predetermined portion of the output signal(s).

41. A method according to embodiment 40, wherein the deriving of the first predetermined portion comprises removing a portion of the output signal(s).

42. A method according to embodiment 41, wherein the removing step comprises removing a portion of the output signal(s) having a frequency below a predetermined threshold frequency.

43. A method according to any of embodiments 39-42, further comprising an element estimating a movement of the housing, where the controller receives an output of the element and determines the parameter also on the basis of the output from the element.

44. A method according to any of embodiments 39-43, wherein the blood perfused part is part of an ear canal of the person or animal, and wherein the housing is provided in the ear canal.

45. A method according to any of embodiments 39-44, further comprising fixing the housing relative to the blood perfused part using a fixing element.

46. A system according to any of embodiments 1-10, 21-26 and 33-38, wherein the VCSEL(s) with Integrated Photodetector is/are configured to output radiation capable of travelling into the blood perfused part.

47. A method according to any of embodiments 11-20, 27-32 and 39-45, wherein the VCSEL(s) with Integrated Photodetector output(s) radiation travelling into the blood perfused part.

48. A system according to any of embodiments 1-10, 21-26, 33-38 and 46, wherein the parameter is a velocity of blood in the blood perfused part.

49. A method according to any of embodiments 11-20, 27-32, 39-45 and 47, wherein the parameter is a velocity of blood in the blood perfused part.

50. A system according to any of embodiments 1-10, 21-26, 33-38, 46 and 48, wherein the controller is configured to also determine relative motion between the blood perfused part and the VCSEL(s) with Integrated Photodetector.

51. A method according to any of embodiments 11-20, 27-32, 39-45, 47 and 49, further comprising the step of determining a relative motion between the blood perfused part and the VCSEL(s) with Integrated Photodetector.

## Claims

1. A system for determining a parameter related to blood flow of a blood perfused part of an ear canal, the system comprising:

    - a housing configured to be provided in the ear canal,
    - one or more VCSEL(s) each with Integrated Photodetector and each being provided at or in the housing, the VCSEL(s) with Integrated Photodetector being positioned so as to emit radiation to and receive radiation from a surface of the ear canal and
    - a controller for receiving output signal(s) from the VCSEL(s) with Integrated Photodetector and determine the parameter related to the blood flow.

2. A method of operating the system according to claim 1 for determining a parameter related to blood flow of a blood perfused part of an ear canal, the method comprising:

    - providing the housing configured to be provided in the ear canal,
    - the one or more VCSEL(s) each with Integrated Photodetector emitting radiation to and receiving radiation from a surface of the ear canal and
    - the controller receiving output signal(s) from the VCSEL(s) with Integrated Photodetector and determining the parameter related to the blood flow.

3. A system for determining a parameter related to blood flow of a blood perfused part of a person or animal, the system comprising:

    - a housing,
    - one or more VCSEL(s) each with Integrated Photodetector and each provided at or in the housing, the VCSEL(s) with Integrated Photodetector being positioned so as to emit radiation to and receive radiation from the surface and
    - a controller configured to:

        - receive the output signal(s) from the VCSEL(s) with Integrated Photodetector,
        - determine, from a first predetermined portion of the output signal(s). the parameter related to the blood flow, and
        - determine, from a second predetermined portion of the output signal(s), a relative movement between the blood perfused part and the VCSEL(s) with Integrated Photodetector.

4. A method of operating the system according to claim 3 for determining a parameter related to blood flow of a blood perfused part of a person or animal, the system comprising:

    - providing the housing,
    - the one or more VCSELs each with Integrated photodetector emitting radiation to and receiving radiation from the surface and
    - the controller:

        - receiving output signal(s) from the VCSEL(s) with Integrated Photodetector,
- deriving a first predetermined portion of the output signal(s) and determining, based thereon, the parameter, and
- deriving a second predetermined portion of the output signal(s) and determining, based thereon, a relative movement between the blood perfused part and the VCSEL(s) with Integrated Photodetector.

5. A system for determining a parameter related to blood flow of a blood perfused part of a person or animal, the system comprising:

- a housing,
- one or more VCSELs each with Integrated Photodetector each being provided at or in the housing, the VCSEL(s) with Integrated Photodetector being positioned so as to emit radiation to and receive radiation from the blood perfused part and
- a controller for receiving output signal(s) from the VCSEL(s) with Integrated Photodetector and determine the parameter, from the output of the VCSEL(s) with Integrated Photodetector, based on a Doppler shift of the radiation received vis-à-vis the radiation emitted, where the Doppler shift is caused by the blood flow.

6. A method of controlling a system according to claim 5 for determining a parameter related to blood flow of a blood perfused part of a person or animal, the system comprising:

- providing the housing,
- the one or more VCSELs each with Integrated Photodetector emitting radiation to and receiving radiation from the blood perfused part and
- the controller for receiving output signal(s) from the VCSEL(s) with Integrated Photodetector and determining the parameter, from the output of the VCSEL(s) with Integrated Photodetector, based on a Doppler shift of the radiation received vis-à-vis the radiation emitted, where the Doppler shift is caused by the blood flow.

7. A system according to any of claims 1, 3 and 5, wherein the VCSEL(s) with Integrated Photodetector is/are configured to output radiation capable of travelling into the blood perfused part.

8. A method according to any of claims 2, 4 and 6, wherein the VCSEL(s) with Integrated Photodetector output(s) radiation travelling into the blood perfused part.

9. A system according to any of claims 1, 3, 5 and 7, wherein the parameter is a velocity of blood in the blood perfused part.

10. A method according to any of claims 2, 4, 6 and 8, wherein the parameter is a velocity of blood in the blood perfused part.

11. A system according to any of claims 1, 3, 5, 7 and 9, wherein the controller is configured to also determine relative motion between the blood perfused part and the VCSEL(s) with Integrated Photodetector.

12. A method according to any of claims 2, 4, 6, 8 and 10, further comprising the step of determining a relative motion between the blood perfused part and the VCSEL(s) with Integrated Photodetector.

Figure 1

Figure 2

Figure 3

Figure 4

EP 4 309 573 A1

Figure 7

Figure 9

Figure 6

Figure 10

Figure 5

Figure 8

EP 4 309 573 A1

160

12

13

240

16    201

14          10

202

41

250

150/152

22

23

Figure 11

160

12

240

14          202

201

41

250

150/152

23

Figure 12

```
        ┌─────┐      ┌─────┐      ┌─────┐      ┌─────┐      ┌─────┐      ┌─────┐
        │ 160 │ ───▶ │  14 │ ───▶ │ 203 │ ───▶ │ 204 │ ───▶ │ 205 │ ───▶ │ 206 │
        └─────┘      └─────┘      └─────┘      └─────┘      └─────┘      └─────┘
```

150/152

Figure 13

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/073378 A1 (WONG MING YIP WALLACE [CN]) 14 April 2022 (2022-04-14) * figures 19-22a * * page 17, line 29 - page 20, line 40 * ----- | 1,2,7-12 | INV. A61B5/026 A61B5/00 |
| X | JP 5 185265 B2 (PIONEER ELECTRONIC CORP) 17 April 2013 (2013-04-17) * paragraph [0001] * * paragraphs [0028] - [0033]; figure 5 * * paragraphs [0086] - [0105]; figures 18-23 * ----- | 1,2,7-12 | |
| A | US 2021/267464 A1 (HIRANO ASAO [JP] ET AL) 2 September 2021 (2021-09-02) * figures 6-12 * * paragraphs [0044] - [0048] * ----- | 1,2,7-12 | |
| A | US 2020/359907 A1 (KASHIWASE SUSUMU [JP]) 19 November 2020 (2020-11-19) * figures 3,4 * * paragraphs [0023] - [0029], [0046] * ----- | 1,2,7-12 | |
| | | | TECHNICAL FIELDS SEARCHED (IPC) |
| X | US 2010/030088 A1 (CARNEY JAMES KEVIN [US] ET AL) 4 February 2010 (2010-02-04) * figures 1-3 * * paragraphs [0039] - [0041], [0050] - [0054], [0068] - [0085] * ----- | 3,4,7-12 | A61B |
| X | US 2021/186345 A1 (ITO ATSUSHI [JP] ET AL) 24 June 2021 (2021-06-24) * figure 2 * * paragraphs [0064] - [0083], [0092] - [0097] * ----- | 3,4,7-12 | |

-/--

The present search report has been drawn up for all claims

| Place of search | Date of completion of the search | Examiner |
|---|---|---|
| The Hague | 13 April 2023 | Dhervé, Gwenaëlle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

| | Application Number |
|---|---|
| | EP 22 18 6284 |

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/234590 A1 (AKKERMANS ANTONIUS HERMANUS MARIA [NL] ET AL) 25 September 2008 (2008-09-25) * figures 1,2 * * paragraphs [0025] – [0030], [0033] * ----- | 5-12 | |
| X | US 2020/323438 A1 (SAWADA RENSHI [JP] ET AL) 15 October 2020 (2020-10-15) * figures 3,7 * * paragraphs [0053] – [0059], [0082] – [0099] * ----- | 5-12 | |
| X | WO 2020/246258 A1 (FUJIFILM CORP [JP]; FUJI XEROX CO LTD [JP]) 10 December 2020 (2020-12-10) * figures 1,2,6,7 * * paragraphs [0007], [0028] – [0033], [0052] – [0059] * ----- | 5-12 | |
| | | | **TECHNICAL FIELDS SEARCHED (IPC)** |

The present search report has been drawn up for all claims

| Place of search | Date of completion of the search | Examiner |
|---|---|---|
| The Hague | 13 April 2023 | Dhervé, Gwenaëlle |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ...................................................................
& : member of the same patent family, corresponding
    document

EPO FORM 1503 03.82 (P04C01)

Europäisches
Patentamt

European
Patent Office

Office européen
des brevets

Application Number

**EP 22 18 6284**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**
**SHEET B**

Application Number

EP 22 18 6284

The Search Division considers that the present European patent application does not comply with the requirements of unity of invention and relates to several inventions or groups of inventions, namely:

    1. claims: 1, 2, 7-12

        Determination of a parameter related to blood flow of a
        blood perfused part of the ear canal.
                        ---


    2. claims: 3, 4, 7-12

        Determination of a parameter related to blood flow of a
        blood perfused part of a person or an animal and of a
        relative movement of the blood perfused part.
                        ---


    3. claims: 5-12

        Determination of a parameter related to blood flow of a
        blood perfused part of a person or an animal based on a
        detected Doppler shift.
                        ---

## ANNEX TO THE EUROPEAN SEARCH REPORT
## ON EUROPEAN PATENT APPLICATION NO.

EP 22 18 6284

This annex lists the patent family members relating to the patent documents cited in the above-mentioned European search report.
The members are as contained in the European Patent Office EDP file on
The European Patent Office is in no way liable for these particulars which are merely given for the purpose of information.

13-04-2023

| Patent document cited in search report | Publication date | Patent family member(s) | Publication date |
|---|---|---|---|
| WO 2022073378 A1 | 14-04-2022 | NONE | |
| JP 5185265 B2 | 17-04-2013 | JP 5185265 B2 | 17-04-2013 |
| | | JP WO2009001449 A1 | 26-08-2010 |
| | | WO 2009001449 A1 | 31-12-2008 |
| US 2021267464 A1 | 02-09-2021 | JP 7244539 B2 | 22-03-2023 |
| | | JP WO2020100677 A1 | 24-09-2021 |
| | | US 2021267464 A1 | 02-09-2021 |
| | | WO 2020100677 A1 | 22-05-2020 |
| US 2020359907 A1 | 19-11-2020 | JP 6891074 B2 | 18-06-2021 |
| | | JP 7023403 B2 | 21-02-2022 |
| | | JP 2019037547 A | 14-03-2019 |
| | | JP 2021121355 A | 26-08-2021 |
| | | US 2020359907 A1 | 19-11-2020 |
| | | WO 2019039223 A1 | 28-02-2019 |
| US 2010030088 A1 | 04-02-2010 | EP 2346394 A1 | 27-07-2011 |
| | | US 2010030088 A1 | 04-02-2010 |
| | | WO 2010014415 A1 | 04-02-2010 |
| US 2021186345 A1 | 24-06-2021 | JP 2019076641 A | 23-05-2019 |
| | | US 2021186345 A1 | 24-06-2021 |
| | | WO 2019082688 A1 | 02-05-2019 |
| US 2008234590 A1 | 25-09-2008 | CN 1950027 A | 18-04-2007 |
| | | EP 1744669 A1 | 24-01-2007 |
| | | JP 4772036 B2 | 14-09-2011 |
| | | JP 2007534421 A | 29-11-2007 |
| | | KR 20070004892 A | 09-01-2007 |
| | | TW 200605847 A | 16-02-2006 |
| | | US 2008234590 A1 | 25-09-2008 |
| | | WO 2005104935 A1 | 10-11-2005 |
| US 2020323438 A1 | 15-10-2020 | JP 7103644 B2 | 20-07-2022 |
| | | JP WO2017208645 A1 | 28-03-2019 |
| | | US 2020323438 A1 | 15-10-2020 |
| | | WO 2017208645 A1 | 07-12-2017 |
| WO 2020246258 A1 | 10-12-2020 | JP 7138244 B2 | 15-09-2022 |
| | | JP WO2020246258 A1 | 10-12-2020 |
| | | WO 2020246258 A1 | 10-12-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110105874 A
- US 7018338 B
- US 8979762 B
- US 8157730 B
- US 2010081940 A
- US 2010177300 A
- US 2021196136 A
- US 2021085245 A
- US 5711308 A
- US 2020275216 A
- US 2020370879 A
- US 2011082355 A
- EP 3439550 A
- WO 18029033 A
- US 20080188726 A